# EUROPEAN PATENT SPECIFICATION

(11) **EP 1 379 618 B1**
(45) Date of publication and mention of the grant of the patent: **14.06.2006**
(21) Application number: 01984742.5
(22) Date of filing: 15.11.2001
(51) Int. Cl.: C11D 3/16

(54) **LIGAND AND COMPLEX FOR CATALYTICALLY BLEACHING A SUBSTRATE**
LIGANDEN UND KOMPLEXE FÜR DIE KATALYTISCHE BLEICHUNG VON SUBSTRATEN
LIGAND ET COMPLEXE DE BLANCHIMENT D'UN SUBSTRAT PAR VOIE CATALYTIQUE

(30) Priority: 15.12.2000 GB 0030673
(43) Date of publication of application: 14.01.2004
(73) Proprietor: UNILEVER PLC, London EC4P 4BQ (GB); UNILEVER N.V., 3013 AL Rotterdam (NL)
(72) Inventor: BOERZEL, Heidi, D-68161, Mannheim (DE); COMBA, Peter, University of Heidelberg, 69120 Heidelberg (DE); HAGE, Ronald, Unilever Research Vlaardingen, NL-3133 AT Vlaardingen (NL); KERSCHER, Marion, University of Heidelberg, 69120 Heidelberg (DE); LIENKE, Joachim, Unilever Research Vlaardingen, NL-3133 AT Vlaardingen (NL); MERZ, Michael, University of Heidelberg, 69120 Heidelberg (DE)
(74) Representative: Elliott, Peter William
(86) International application number: PCT/EP2001/013314
(87) International publication number: WO 2002/048301

(56) References cited:
- WO-A-00/60045
- R. HALLER: "Zur Kenntnis substituierter 3,7-Diaza- und 3-Oxa-7-aza-bicyclo-[3.3.1]-nonanone" ARZNEIMITTEL FORSCH., vol. 15, no. 11, 1965, pages 1327-1330, XP002196150
- U. KUHL ET AL.: "Diazabicyclo[3.3.1]nonanone-type Ligands for the Opioid Receptors" ARCH. PHARM., vol. 333, no. 7, 2000, pages 226-230, XP002196151
- H. BÖRZEL ET AL.: "Stabilization of Copper Dioxygen Compounds: Design, Synthesis, and Characterization" CHEM. EUR. J., vol. 5, no. 6, 1999, pages 1716-1721, XP002196152 cited in the application

## Description

### FIELD OF INVENTION

This invention relates to a class of ligand or complex thereof useful as catalysts for catalytically bleaching substrates with atmospheric oxygen.

### BACKGROUND OF INVENTION

The use of bleaching catalysts for stain removal has been developed over recent years. The recent discovery that some catalysts are capable of bleaching effectively in the absence of an added peroxyl source has recently become the focus of some interest, for example: WO9965905; W00012667; W00012808; and, WO0029537.

The search for new classes of compounds that are suitable as air bleaching catalyst is ongoing.

Various [3.3.1] bicyclo compounds and complexes thereof are discussed in the literature, see for example: Comba P. et al., J. Chem. Soc. Dalton Trans, 1998, (23) 3997-4001; Börzel et al. Chem. Eur. J. 1999, 5, No. 6, 1716 to 1721 and review by P. Comba in Coordination Chemistry Reviews 2000, 200-202, 217 to 245, entitled "Coordination compounds in the Entactic State". These compounds are discussed in terms of their physical properties. 2,4-bis(2-pyridyl)-3,7-diazabicyclo[3.3.1]nonane ligands are disclosed in Haller R., Arzneimittel Forsch., 1965, 15(11), 1327-1330.

WO0060045 discloses a bleaching system comprising: a) from about 1ppb, by weight of a transition metal catalyst comprising: i) a transition metal; ii) a ligand having formula (I): 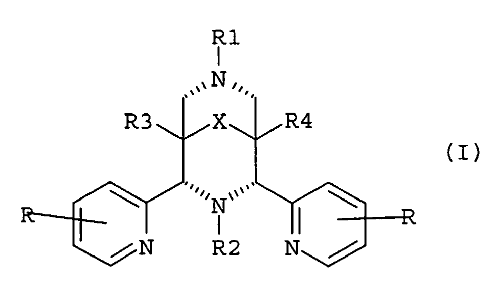 wherein each R is independently hydrogen, hydroxyl, C1-C4 alkyl, and mixtures thereof; R1 is C1-C4 alkyl, C6-C10 aryl, and mixtures thereof; R2 is C1-C4 alkyl, C6-C10 aryl, and mixtures thereof; R3 and R4 are each independently hydrogen, C1-C8 alkyl, C1-C8 hydroxyalkyl, -(CH₂)ₓCO₂R5 wherein R5 is C1-C4 alkyl, x is from 0 to 4, and mixtures thereof; X is carbonyl, -C(R6)2- wherein each R6 is independently hydrogen,
hydroxyl, C1-C4 alkyl, and mixtures thereof; b) optionally a source of hydrogen peroxide; and c) the balance carriers and adjunct ingredients. However, the teaching of W00060045 limits substituents at the nitrogens (3 and 7 positions) of bicyclostructure to homoaromatic carbon groups, namely alkyl and aryl.

### SUMMARY OF INVENTION

We have found that the presence of a group bearing a heteroatom on one or more of the nitrogens of the bicyclostructure provides an enhanced activity. The compounds provided are surprisingly active as air bleaching catalysts. In addition, we also found that similar compounds are surprisingly active and provide novel ligands and transition metal complexes thereof for use in air bleaching.

Accordingly, in a first aspect, the present invention provides a bleaching composition comprising:

A bleaching composition comprising:
a) a monomer ligand or transition metal catalyst thereof of a ligand having the formula (I): 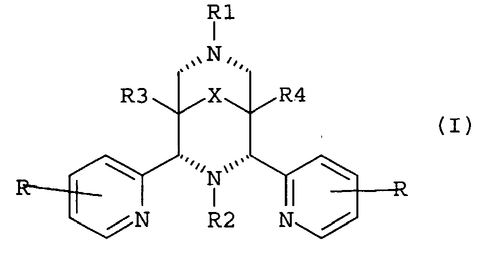 wherein each R is independently selected from: hydrogen, F, Cl, Br, hydroxyl, C1-C4-alkylO-, -NH-CO-H, -NH-CO-C1-C4-alkyl, -NH2, -NH-C1-C4-alkyl, and C1-C4-alkyl;
   R1 and R2 are independently selected from:
   C1-C4-alkyl,
   C6-C10-aryl, and,
   a group containing a heteroatom capable of coordinating to a transition metal, wherein at least one of R1 and R2 is the group containing the heteroatom;
   R3 and R4 are independently selected from hydrogen, C1-C8 alkyl, C1-C8-alkyl-O-C1-C8-alkyl, C1-C8-alkyl-O-C6-C10-aryl, C6-C10-aryl, C1-C8-hydroxyalkyl, and -(CH2)ₙC(O)OR5
   wherein R5 is independently selected from: hydrogen, C1-C4-alkyl, n is from 0 to 4, and mixtures thereof; and,
   X is selected from C=O, -[C(R6)₂]_{y}- wherein Y is from 0 to 3 each R6 is independently selected from hydrogen, hydroxyl, C1-C4-alkoxy and C1-C4-alkyl; and,
b) the balance carriers and adjunct ingredients.

Preferred groups containing the heteroatom may be found in a heterocycloalkyl: selected from the group consisting of:
pyrrolinyl; pyrrolidinyl; morpholinyl; piperidinyl; piperazinyl; hexamethylene imine; 1,4-piperazinyl; tetrahydrothiophenyl; tetrahydrofuranyl; tetrahydropyranyl; and oxazolidinyl, wherein the heterocycloalkyl may be connected to the ligand via any atom in the ring of the selected heterocycloalkyl,
a -C1-C6-alkyl-heterocycloalkyl, wherein the heterocycloalkyl of the -C1-C6-heterocycloalkyl is selected from the group consisting of: piperidinyl; piperidine; 1,4-piperazine,tetrahydrothiophene; tetrahydrofuran; pyrrolidine; and tetrahydropyran, wherein the heterocycloalkyl may be connected to the -C1-C6-alkyl via any atom in the ring of the selected heterocycloalkyl,
a -C1-C6-alkyl-heteroaryl, wherein the heteroaryl of the - C1-C6-alkylheteroaryl is selected from the group consisting of: pyridinyl; pyrimidinyl; pyrazinyl; triazolyl; pyridazinyl; 1,3,5-triazinyl; quinolinyl; isoquinolinyl; quinoxalinyl; imidazolyl; pyrazolyl; benzimidazolyl; thiazolyl; oxazolidinyl; pyrrolyl; carbazolyl; indolyl; and
isoindolyl, wherein the heteroaryl may be connected to the - C1-C6-alkyl via any atom in the ring of the selected heteroaryl and the selected heteroaryl is optionally substituted by -C1-C4-alkyl,
a -C0-C6-alkyl-phenol or thiophenol,
a -C2-C4-alkyl-thiol, thioether or alcohol,
a -C2-C4-alkyl-amine, and
a -C2-C4-alkyl-carboxylate.

In a second aspect, the present invention provides a bleaching composition comprising, in an aqueous medium, atmospheric oxygen and a bicyclo ligand of the general Formula (I) which forms a complex with a transition metal, the complex catalysing bleaching of a substrate by the atmospheric oxygen, wherein the aqueous medium is substantially devoid of peroxygen bleach or a peroxy-based or -generating bleach system. It is preferred that the medium has a pH value in the range from pH 6 to 11 and most preferably from pH 8 to 10.

The present invention also provides novel compounds of the general Formula (I) with the proviso that the following compounds are excluded:
dimethyl 2,4-di-(2-pyridyl)-3,7-bis-(pyridin-2-ylmethyl)-3,7-diaza-bicyclo[3.3.1]nonan-9-one-1,5-dicarboxylate;
1,5-bis-(hydroxymethylene)-2,4-di-(2-pyridyl)-3,7-bis-(pyridin-2-ylmethyl)-3,7-diazabicyclo[3.3.1]nonan-9-ol;
dimethyl 2,4-di-(2-pyridyl)-3,7-bis-(pyridin-2-ylethyl)-3,7-diaza-bicyclo[3.3.1]nonan-9-one-1,5-dicarboxylate;
dimethyl 2,4-di-(2-pyridyl)-3-(5-carboxypentyl)-7-methyl-3,7-diaza-bicyclo[3.3.1]nonan-9-one-1,5-dicarboxylate;
dimethyl 2,4-di-(2-pyridyl)-3-(2-methoxyethyl)-7-methyl-3,7-diaza-bicyclo[3.3.1]nonan-9-one-1,5-dicarboxylate; diethyl-2,4-dipyridyl-7-picolyl-3,7-diaza-bicyclo-[3.3.1]-nonan-9-one-1,5-dicarboxylate; diethyl-2,4-dipyridyl-7-benzyl-3-hydroxyethyl-3,7-diaza-bicyclo-[3.3.1]-nonan-9-one-1,5-dicarboxylate; and, dimethyl-2,4-dipyridyl-7-benzyl-3-hydroxyethyl-3,7-diaza-bicyclo-[3.3.1]-nonan-9-one-1,5-dicarboxylate.

An advantage of the class of ligand and complex according to the present invention is that the complex can catalyse bleaching of a substrate by atmospheric oxygen, thus permitting its use in a medium such as an aqueous medium that is substantially devoid of peroxygen bleach or a peroxy-based or -generating bleach system. We have also found that complexes of this class are surprisingly effective in catalysing bleaching of the substrate by atmospheric oxygen after treatment of the substrate. The composition of the present invention bleaches a substrate with at least 10 %, preferably at least 50 % and optimally at least 90 % of any bleaching of the substrate being effected by oxygen sourced from the air.

One skilled in the art will appreciate that not all peroxyl activating catalysts are capable of functioning as an oxygen activation catalyst. However, the converse is not true. There is no evidence to indicate that any oxygen activation catalyst will not function as peroxyl activating catalyst. In this regard, all oxygen activation catalysts disclosed herein may be used as a peroxyl activating catalyst. Catalysts of the present invention may be incorporated into a composition together with a peroxyl species or source thereof. For a discussion of acceptable ranges of a peroxyl species or source thereof and other adjuvants that may be present the reader is directed to United States Patent 6,022,490, the contents of which are incorporated by reference.

The present invention extends to a method of bleaching a substrate comprising applying to the substrate, in an aqueous medium, the bleaching composition according to the present invention.

The present invention extends to a commercial package comprising the bleaching composition according to the present invention together with instructions for its use.

The present invention further provides a dry textile having an organic substance as defined above applied or deposited thereon, whereby bleaching by atmospheric oxygen is catalysed on the textile.

Advantageously, by enabling a bleaching effect even after the textile has been treated, the benefits of bleaching can be prolonged on the textile. Furthermore, since a bleaching effect is conferred to the textile after the treatment, the treatment itself, such as a laundry wash cycle, may for example be shortened. Moreover, since a bleaching effect is achieved by atmospheric oxygen after treatment of the textile, hydrogen peroxide or peroxy-based bleach systems can be omitted from the treatment substance.

The organic substance may be contacted to the textile fabric in any suitable manner. For example, it may be applied in dry form, such as in powder form, or in a liquor that is then dried, for example as an aqueous spray-on fabric treatment fluid or a wash liquor for laundry cleaning, or a non-aqueous dry cleaning fluid or spray-on aerosol fluid. Other suitable means of contacting the organic substance to the textile may be used, as further explained below.

Any suitable textile that is susceptible to bleaching or one that one might wish to subject to bleaching may be used. Preferably the textile is a laundry fabric or garment.

In a preferred embodiment, the method according to the present invention is carried out on a laundry fabric using an aqueous treatment liquor. In particular, the treatment may be effected in a wash cycle for cleaning laundry. More preferably, the treatment is carried out in an aqueous detergent bleach wash liquid.

In a preferred embodiment, the treated textile is dried, by allowing it to dry under ambient temperature or at elevated temperatures. The elevated temperatures are commonly provided by a heated agitated environment, as for example found in a tumble dryer, which has been found to accelerate and enhance the air bleaching effect.

The bleaching method may be carried out by simply leaving the substrate in contact with the organic substance for a sufficient period of time. Preferably, however, the organic substance is in an aqueous medium, and the aqueous medium on or containing the substrate is agitated.

The organic substance can be contacted with the textile fabric in any conventional manner. For example it may be applied in dry form, such as in powder form, or in a liquor that is then dried, for example in an aqueous spray-on fabric treatment fluid or a wash liquor for laundry cleaning, or a non-aqueous dry cleaning fluid or spray-on aerosol fluid.

In a preferred embodiment, the treated textile is dried, by allowing it to dry under ambient temperature or at elevated temperatures.

In a particularly preferred embodiment the method according to the present invention is carried out on a laundry fabric using aqueous treatment liquor. In particular the treatment may be effected in, or as an adjunct to, an essentially conventional wash cycle for cleaning laundry. More preferably, the treatment is carried out in an aqueous detergent wash liquor. The organic substance can be delivered into the wash liquor from a powder, granule, pellet, tablet, block, bar or other such solid form. The solid form can comprise a carrier, which can be particulate, sheet-like or comprise a three-dimensional object. The carrier can be dispersible or soluble in the wash liquor or may remain substantially intact. In other embodiments, the organic substance can be delivered into the wash liquor from a paste, gel or liquid concentrate.

It is particularly advantageous that the organic substance used in the method of the present invention makes use of atmospheric oxygen in its bleaching activity. This avoids the requirement that peroxygen bleaches and/or other relatively large quantities of reactive substances need be used in the treatment process. Consequently, only a relatively small quantity of bleach active substance need be employed and this allows dosage routes to be exploited that could previously not be used. Thus, while it is preferable to include the organic substance in a composition that is normally used in a washing process, such as a pre-treatment, main-wash, conditioning composition or ironing aid, other means for ensuring that the organic substance is present in the wash liquor may be envisaged.

For example, it is envisaged that the organic substance can be presented in the form of a body from which it is slowly released during the whole or part of the laundry process. Such release can occur over the course of a single wash or over the course of a plurality of washes. In the latter case it is envisaged that the organic substance can be released from a carrier substrate used in association with the wash process, e.g. from a body placed in the dispenser drawer of a washing machine, elsewhere in the delivery system or in the drum of the washing machine. When used in the drum of the washing machine the carrier can be freely moving or fixed relative to the drum. Such fixing can be achieved by mechanical means, for example by barbs that interact with the drum wall, or employ other forces, for example a magnetic force. The modification of a washing machine to provide for means to hold and retain such a carrier is envisaged similar means being known from the analogous art of toilet block manufacture. Freely moving carriers such as shuttles for dosage of surfactant materials and/or other detergent ingredients into the wash can comprise means for the release of the organic substance into the wash.

In the alternative, the organic substance can be presented in the form of a wash additive that preferably is soluble. The additive can take any of the physical forms used for wash additives, including powder, granule, pellet, sheet, tablet, block, bar or other such solid form or take the form of a paste, gel or liquid. Dosage of the additive can be unitary or in a quantity determined by the user. While it is envisaged that such additives can be used in the main washing cycle, the use of them in the conditioning or drying cycle is not hereby excluded.

The present invention is not limited to those circumstances in which a washing machine is employed, but can be applied where washing is performed in some alternative vessel. In these circumstances it is envisaged that the organic substance can be delivered by means of slow release from the bowl, bucket or other vessel which is being employed, or from any implement which is being employed, such as a brush, bat or dolly, or from any suitable applicator.

Suitable pre-treatment means for application of the organic substance to the textile material prior to the main wash include sprays, pens, roller-ball devices, bars, soft solid applicator sticks and impregnated cloths or cloths containing microcapsules. Such means are well known in the analogous art of deodorant application and/or in spot treatment of textiles. Similar means for application are employed in those embodiments where the organic substance is applied after the main washing and/or conditioning steps have been performed, e.g. prior to or after ironing or drying of the cloth. For example, the organic substance may be applied using tapes, sheets or sticking plasters coated or impregnated with the substance, or containing microcapsules of the substance. The organic substance may for example be incorporated into a drier sheet so as to be activated or released during a tumble-drier cycle, or the substance can be provided in an impregnated or microcapsule-containing sheet so as to be delivered to the textile when ironed.

Many transition metal complexes have high extinction coefficients in the visible. In this regard, use over time may result in some colour deposition on a substrate after repeated washing. The addition of a limited amount of a peroxyl source serves to reduce colour deposition in those instances in which it occurs whilst still permitting air bleaching. Nevertheless, we have found that in certain instances the free ligand may be used in the bleaching composition of the present invention. By using a free ligand, a bleaching formulation may prepared that is consistent with consumer formulation colour expectation. In such a formulation the metal ion may be provided by the composition or by trace metals found in the stain.

### DETAILED DESCRIPTION OF THE INVENTION

The ligand as described herein is capable of dynamic inversion. The ability of the ligand to chelate to a TM depends upon the stereochemistry of the substituents. It is preferred that subsituents are endo-endo, but it is likely that stereochemical conversion takes place by retro-Mannich conversion. Retro-Mannich may be prevented by changing the groups present such that retro-Mannich reactions are unfavoured. Nevertheless, it is likely that endo-exo and exo-exo ligands as described herein coordinate to transition metal ions in many instances and are capable of functioning as air bleaching catalysts.

Referring to ligands and complexes thereof and bleaching compositions derived therefrom with respect to Formula (I), it is preferred that each R is the same; and R3 = R4, and more preferred that R3 and R4 are the same and are - (CH2)ₙC(O)O-C1-C4-alkyl. It is even more preferred that R3 and R4 are selected from the group consisting of -CH2OH, - C(O)O-C1-C6-alkyl, and phenyl.

Referring to X, it is preferred that Y = 1, and most preferred wherein X is C=O.

It is preferred that at least one of R1 and R2 is a 3-C0-C6-alkyl-pyridin-2-yl-C0-C6-alkyl. It is most preferred that at least one of R1 and R2 is selected from the group consisting of: 3-ethyl-pyridin-2-ylmethyl, pyridin-2-ylmethyl, 3-methyl-pyridin-2-ylmethyl, and 6-amide-pyridin-2-ylmethyl, of which pyridin-2-ylmethyl is preferred from this group. It is even more preferred that both R1 and R2 are selected from this group.

The catalyst may be used as a preformed complex of the ligand and a transition metal. Alternatively, the catalyst may be formed from the free ligand that complexes with a transition metal already present in the water or that complexes with a transition metal present in the substrate. The composition may also be formulated as a composition of the free ligand or a transition metal-substitutable metal-ligand complex, and a source of transition metal, whereby the complex is formed *in situ* in the medium.

The ligand forms a complex with one or more transition metals, in the latter case for example as a dinuclear complex. Suitable transition metals include for example: manganese in oxidation states II-V, iron II-V, copper I-III, cobalt I-III, titanium II-IV, tungsten IV-VI, vanadium II-V and molybdenum II-VI.

The ligand forms a complex of the general formula (A1):

[MₐLₖXₙ]Yₘ (A1)

in which:
M represents a metal selected from Mn(II)-(III)-(IV)-(V), Cu(I)-(II)-(III), Fe(II)-(III)-(IV)-(V), Co(I)-(II)-(III), Ti(II)-(III)-(IV), V(II)-(III)-(IV)-(V), Mo(II)-(III)-(IV)-(V)-(VI) and W(IV)-(V)-(VI), preferably selected from Fe(II)-(III)-(IV)-(V);
L represents a ligand as herein defined, or its protonated or deprotonated analogue;
X represents a coordinating species selected from any mono, bi or tri charged anions and any neutral molecules able to coordinate the metal in a mono, bi or tridentate manner, preferably selected from O²⁻, RBO₂²⁻, RCOO⁻, RCONR⁻, OH⁻, NO₃⁻, NO, S²⁻, RS⁻, PO₄³⁻, PO₃OR³⁻, H₂O, CO₃²⁻, HCO₃⁻, ROH, N(R)₃, ROO⁻, O₂²⁻, O₂⁻, RCN, Cl⁻, Br⁻, OCN⁻, SCN⁻, CN⁻, N₃⁻, F⁻, I⁻, RO⁻, ClO₄⁻, and CF₃SO₃⁻, and more preferably selected from O²⁻, RBO₂²⁻, RCOO⁻, OH⁻, NO₃⁻, S²⁻, RS⁻, PO₃⁴⁻, H₂O, CO₃²⁻, HCO₃⁻, ROH, N(R)₃, Cl⁻ , Br⁻, OCN⁻, SCN⁻, RCN, N₃⁻, F⁻, I⁻, RO⁻, ClO₄⁻, and CF₃SO₃⁻;
Y represents any non-coordinated counter ion, preferably selected from ClO₄⁻, BR₄⁻, [MX₄]⁻, [MX₄]²⁻, PF₆⁻, RCOO⁻, NO₃⁻, RO⁻, N⁺(R)₄, ROO⁻, O₂²⁻, O₂⁻, Cl⁻ , Br⁻, F⁻, I⁻, CF₃SO₃⁻, S₂O₆²⁻ , OCN⁻, SCN⁻, H₂O, RBO₂²⁻, BF₄⁻ and BPh₄⁻, and more preferably selected from ClO₄⁻, BR₄⁻ , [FeCl₄] ⁻, PF₆⁻, RCOO⁻, NO₃⁻, RO⁻, N⁺(R)₄, Cl⁻ , Br⁻, F⁻, I⁻, CF₃SO₃⁻, S₂O₆²⁻ , OCN⁻, SCN⁻, H₂O and BF₄⁻;
a represents an integer from 1 to 10, preferably from 1 to 4;
k represents an integer from 1 to 10;
n represents an integer from 1 to 10, preferably from 1 to 4;
m represents zero or an integer from 1 to 20, preferably from 1 to 8; and
each R independently represents a group selected from hydrogen, hydroxyl, -R' and -OR', wherein R'= alkyl, alkenyl, cycloalkyl, heterocycloalkyl, aryl, heteroaryl or a carbonyl derivative group, R' being optionally substituted by one or more functional groups E, wherein E independently represents a functional group selected from -F, -Cl, -Br, -I, -OH, -OR', -NH₂, -NHR', -N(R')₂, -N(R')₃⁺, -C(O)R', -OC(O)R', -COOH, -COO⁻ (Na⁺, K⁺), -COOR', -C(O)NH₂, -C(O)NHR', -C(O)N(R')₂, heteroaryl, -R', -SR', -SH, -P(R')₂, -P(O)(R')₂, -P(O)(OH)₂, -P(O)(OR')₂, -NO₂, -SO₃H, -S0₃⁻(Na⁺, K⁺), -S(O)₂R', -NHC(O)R', and -N(R')C(O)R', wherein R' represents cycloalkyl, aryl, arylalkyl, or alkyl optionally substituted by -F, -Cl, -Br, -I, -NH₃⁺, -SO₃H, -SO₃⁻(Na⁺, K⁺), -COOH, -COO⁻ (Na⁺, K⁺), - P(O)(OH)₂, or -P(O)(O⁻ (Na⁺, K⁺))₂, and preferably each R independently represents hydrogen, optionally substituted alkyl or optionally substituted aryl, more preferably hydrogen or optionally substituted phenyl, naphthyl or C₁₋₄₋alkyl.

The counter ions Y in formula (Al) balance the charge z on the complex formed by the ligand L, metal M and coordinating species X. Thus, if the charge z is positive, Y may be an anion such as RCOO⁻, BPh₄⁻, ClO₄⁻, BF₄⁻, PF₆⁻, RSO₃⁻, RSO₄⁻, SO₄²⁻, NO₃⁻, F⁻, Cl⁻, Br⁻, or I⁻, with R being hydrogen, optionally substituted alkyl or optionally substituted aryl. If z is negative, Y may be a common cation such as an alkali metal, alkaline earth metal or (alkyl)ammonium cation.

Suitable counter ions Y include those which give rise to the formation of storage-stable solids. Preferred counter ions for the preferred metal complexes are selected from R⁷COO⁻, ClO₄⁻, BF₄⁻, PF₆⁻, RSO₃⁻ (in particular CF₃SO₃⁻), RSO₄⁻, SO₄²⁻, NO₃⁻, F⁻, Cl⁻, Br⁻, and I⁻, wherein R represents hydrogen or optionally substituted phenyl, naphthyl or C₁-C₄ alkyl.

The novel compounds of Formula (I) as provided by the present invention also extend to their various transition metal complexes, the transition metal complexes are as discussed above with reference to (A1).

It will be appreciated that the complex (A1) can be formed by any appropriate means, including *in situ* formation whereby precursors of the complex are transformed into the active complex of general formula (A1) under conditions of storage or use. Preferably, the complex is formed as a well-defined complex or in a solvent mixture comprising a salt of the metal M and the ligand L or ligand L-generating species. Alternatively, the catalyst may be formed *in situ* from suitable precursors for the complex, for example in a solution or dispersion containing the precursor materials. In one such example, the active catalyst may be formed in situ in a mixture comprising a salt of the metal M and the ligand L, or a ligand L-generating species, in a suitable solvent. Thus, for example, if M is iron, an iron salt such as FeSO₄ can be mixed in solution with the ligand L, or a ligand L-generating species, to form the active complex. Thus, for example, the composition may formed from a mixture of the ligand L and a metal salt MXₙ in which preferably n=1-5, more preferably 1-3. In another such example, the ligand L, or a ligand L-generating species, can be mixed with metal M ions present in the substrate or wash liquor to form the active catalyst in situ. Suitable ligand L-generating species include metal-free compounds or metal coordination complexes that comprise the ligand L and can be substituted by metal M ions to form the active complex according the formula (A1).

The catalysts according to the present invention may be used for laundry cleaning, hard surface cleaning (including cleaning of lavatories, kitchen work surfaces, floors, mechanical ware washing etc.). As is generally known in the art, bleaching compositions are also employed in waste-water treatment, pulp bleaching during the manufacture of paper, leather manufacture, dye transfer inhibition, food processing, starch bleaching, sterilisation, whitening in oral hygiene preparations and/or contact lens disinfection.

In typical washing compositions the level of the organic substance is such that the in-use level is from 1µM to 50mM, with preferred in-use levels for domestic laundry operations falling in the range 10 to 100 µM. Higher levels may be desired and applied in industrial bleaching processes, such as textile and paper pulp bleaching. These levels reflect the amount of catalyst that may be present in a wash dose of a detergent composition. The bleaching composition comprises at least 1 ppb of the ligand or complex thereof.

In the context of the present invention, bleaching should be understood as relating generally to the decolourisation of stains or of other materials attached to or associated with a substrate. However, it is envisaged that the present invention can be applied where a requirement is the removal and/or neutralisation by an oxidative bleaching reaction of malodours or other undesirable components attached to or otherwise associated with a substrate. Furthermore, in the context of the present invention bleaching is to be understood as being restricted to any bleaching mechanism or process that does not require the presence of light or activation by light.

### Synthesis

In addition to the utility of the ligands and complexes of the present invention as catalysts another advantage is that the ligands are generally relatively easy to synthesise in comparison to other ligands. The following is one example of a strategic synthetic approach; it will be evident to one skilled in the art of synthetic organic chemistry that many approaches may be taken to obtain ligands and complexes for use in the present invention. The ease of synthesis of the ligand of Formula (I) is dependent upon the nature of substituents about the structure. The ligands of Formula (I) are most preferably symmetric. Synthesis of these types of molecules are found in articles by U. Holzgrabe et al. in Arch. Pharm. (Weinheim, Ger.) 1992, 325, 657 and A. Samhammer et al. Arch. Pharm. (Weinheim, Ger.) 1984, 322, 557. Below is given a schematic example illustrating the ease of synthesis. The synthesis is shown in a two step synthesis, Scheme 1 and Scheme 2, but in some cases may be conducted as a "one-pot" synthesis depending upon the nature of the substituents. Nevertheless, where substituents R7 = R8 are different from R3 = R4 a two step synthesis is preferred. The product of reaction as found in Scheme 1 is referred to as dimethyl 2,6-di-(2-pyridyl)-1-methyl-piperid-4-one-3,5-dicarboxylate (NPy2), which can easily tautomerize to the enol. The synthesis is exemplified in R. Haller, K.W. Merz, Pharm. Acta Helv., **1963**, 442.

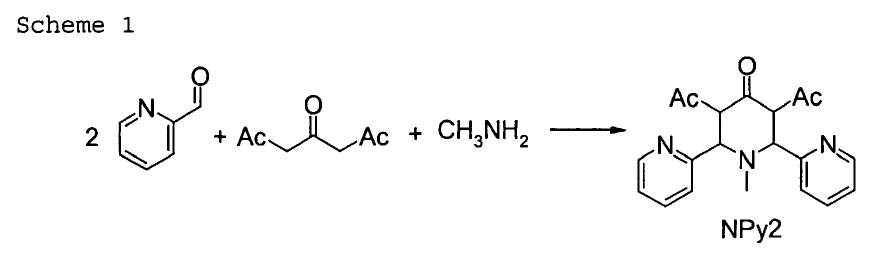

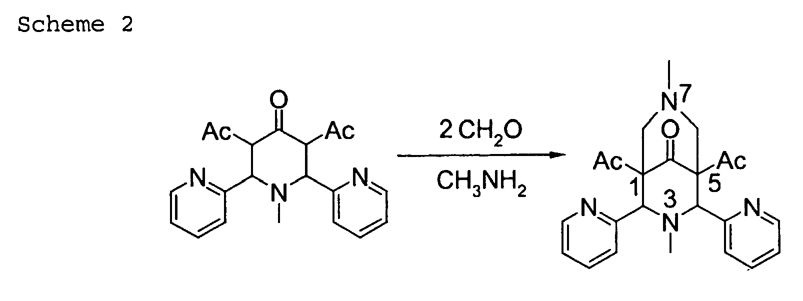

Another important intermediate that may be produced according to the general teachings of Scheme 1 wherein methylamine (CH₃NH₂) is replaced by 2-aminomethyl-pyridine such that a product referred to as dimethyl 2,6-di-(2-pyridyl)-1-(pyridin-2-ylmethyl)-piperid-4-one-3,5-dicarboxylate (NPy3) is produced, the structure of which is given below.

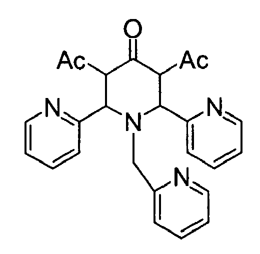

One skilled in the art will appreciate that whilst Ac [-CO(O)Me] is an electron withdrawing group and electron withdrawing groups are generally preferred to facilitate synthesis other groups will also allow the reaction to proceed. Examples of suitable electron withdrawing groups are given above and will be evident to one skilled in the art. The reaction is also driven by precipitation of the product from solution.

In instances, depending upon the nature of the substituents, for example a phenolic group, it will be necessary to protect certain functional groups. The choice of protecting groups during synthesis to prevent undesirable reactions will be evident to one skilled in the art. For a discussion of protecting groups in organic synthesis the reader is directed to T. W. Green and P. G. M. Wuts, Protective Groups In Organic Synthesis 3nd Ed.; J. Wiley and Sons, 1999.

It will be evident that if a diamine is substituted for methylamine in the reaction illustrated in Scheme 2 two structures may be linked together via the 7 positions as found in the structure below.

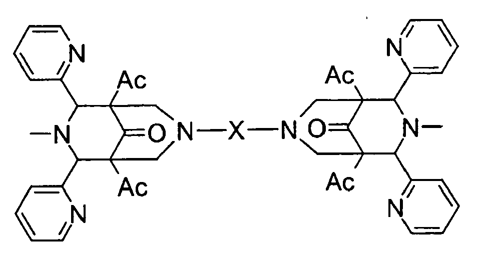

In addition, if a diamine is substituted for methylamine in the reaction illustrated in Scheme 1 a NPy2 structure is formed that is linked at the 3 positions. Obviously, this dimer would serve as a precursor to other dimer and polymer type structures. The present invention is confined to "monomer" ligands and not the dimer and polymer units linked by a covalent bond as described above. The term "monomer" as used herein is used to exclude these products in which covalently linked polyligand type structures are formed.

### The Detergent Composition.

The air bleach catalyst and may be used in a detergent composition specifically suited for stain bleaching purposes, and this constitutes a second aspect of the invention. To that extent, the composition comprises a surfactant and optionally other conventional detergent ingredients. The invention in its second aspect provides an enzymatic detergent composition which comprises from 0.1 - 50 % by weight, based on the total detergent composition, of one or more surfactants. This surfactant system may in turn comprise 0 - 95 % by weight of one or more anionic surfactants and 5 to 100 % by weight of one or more nonionic surfactants. The surfactant system may additionally contain amphoteric or zwitterionic detergent compounds, but this in not normally desired owing to their relatively high cost. The enzymatic detergent composition according to the invention will generally be used as a dilution in water of about 0.05 to 2%.

In general, the nonionic and anionic surfactants of the surfactant system may be chosen from the surfactants described "Surface Active Agents" Vol. 1, by Schwartz & Perry, Interscience 1949, Vol. 2 by Schwartz, Perry & Berch, Interscience 1958, in the current edition of "McCutcheon's Emulsifiers and Detergents" published by Manufacturing Confectioners Company or in "Tenside-Taschenbuch", H. Stache, 2nd Edn., Carl Hauser Verlag, 1981.

Suitable nonionic detergent compounds which may be used include, in particular, the reaction products of compounds having a hydrophobic group and a reactive hydrogen atom, for example, aliphatic alcohols, acids, amides or alkyl phenols with alkylene oxides, especially ethylene oxide either alone or with propylene oxide. Specific nonionic detergent compounds are C₆-C₂₂ alkyl phenol-ethylene oxide condensates, generally 5 to 25 EO, i.e. 5 to 25 units of ethylene oxide per molecule, and the condensation products of aliphatic C₈₋C₁₈ primary or secondary linear or branched alcohols with ethylene oxide, generally 5 to 40 EO.

Suitable anionic detergent compounds which may be used are usually water-soluble alkali metal salts of organic sulphates and sulphonates having alkyl radicals containing from about 8 to about 22 carbon atoms, the term alkyl being used to include the alkyl portion of higher acyl radicals. Examples of suitable synthetic anionic detergent compounds are sodium and potassium alkyl sulphates, especially those obtained by sulphating higher C₈-C₁₈ alcohols, produced for example from tallow or coconut oil, sodium and potassium alkyl C₉-C₂₀ benzene sulphonates, particularly sodium linear secondary alkyl C₁₀-C₁₅ benzene sulphonates; and sodium alkyl glyceryl ether sulphates, especially those ethers of the higher alcohols derived from tallow or coconut oil and synthetic alcohols derived from petroleum. The preferred anionic detergent compounds are sodium C₁₁-C₁₅ alkyl benzene sulphonates and sodium C₁₂-C₁₈ alkyl sulphates. Also applicable are surfactants such as those described in EP-A-328 177 (Unilever), which show resistance to salting-out, the alkyl polyglycoside surfactants described in EP-A-070 074, and alkyl monoglycosides.

Preferred surfactant systems are mixtures of anionic with nonionic detergent active materials, in particular the groups and examples of anionic and nonionic surfactants pointed out in EP-A-346 995 (Unilever). Especially preferred is surfactant system that is a mixture of an alkali metal salt of a C₁₆-C₁₈ primary alcohol sulphate together with a C₁₂-C₁₅ primary alcohol 3-7 EO ethoxylate.

The nonionic detergent is preferably present in amounts greater than 10%, e.g. 25-90% by weight of the surfactant system. Anionic surfactants can be present for example in amounts in the range from about 5% to about 40% by weight of the surfactant system.

The detergent composition may take any suitable physical form, such as a powder, granular composition, tablets, a paste or an anhydrous gel.

### Enzymes

The detergent compositions of the present invention may additionally comprise one or more enzymes, which provide cleaning performance, fabric care and/or sanitation benefits.

Said enzymes include oxidoreductases, transferases, hydrolases, lyases, isomerases and ligases. Suitable members of these enzyme classes are described in Enzyme nomenclature 1992: recommendations of the Nomenclature Committee of the International Union of Biochemistry and Molecular Biology on the nomenclature and classification of enzymes, 1992, ISBN 0-12-227165-3, Academic Press.

Examples of the hydrolases are carboxylic ester hydrolase, thiolester hydrolase, phosphoric monoester hydrolase, and phosphoric diester hydrolase which act on the ester bond; glycosidase which acts on O-glycosyl compounds; glycosylase hydrolysing N-glycosyl compounds; thioether hydrolase which acts on the ether bond; and exopeptidases and endopeptidases which act on the peptide bond. Preferable among them are carboxylic ester hydrolase, glycosidase and exo- and endopeptidases. Specific examples of suitable hydrolases include (1) exopeptidases such as aminopeptidase and carboxypeptidase A and B and endopeptidases such as pepsin, pepsin B, chymosin, trypsin, chymotrypsin, elastase, enteropeptidase, cathepsin B, papain, chymopapain, ficain, thrombin, plasmin, renin, subtilisin, aspergillopepsin, collagenase, clostripain, kallikrein, gastricsin, cathepsin D, bromelain, chymotrypsin C, urokinase, cucumisin, oryzin, proteinase K, thermomycolin, thermitase, lactocepin, thermolysin, bacillolysin. Preferred among them is subtilisin; (2) glycosidases such as α-amylase, β-amylase, glucoamylase, isoamylase, cellulase, endo-1,3(4)-β-glucanase (β-glucanase), xylanase, dextranase, polygalacturonase (pectinase), lysozyme, invertase, hyaluronidase, pullulanase, neopullulanase, chitinase, arabinosidase, exocellobiohydrolase, hexosaminidase, mycodextranase, endo-1,4-β-mannanase (hemicellulase), xyloglucanase, endo-β-galactosidase (keratanase), mannanase and other saccharide gum degrading enzymes as described in WO-A-99/09127. Preferred among them are α-amylase and cellulase; (3) carboxylic ester hydrolase including carboxylesterase, lipase, phospholipase, pectinesterase, cholesterol esterase, chlorophyllase, tannase and wax-ester hydrolase. Preferred among them is lipase.

Examples of transferases and ligases are glutathione S-transferase and acid-thiol ligase as described in WO-A-98/59028 and xyloglycan endotransglycosylase as described in WO-A-98/38288.

Examples of lyases are hyaluronate lyase, pectate lyase, chondroitinase, pectin lyase, alginase II. Especially preferred is pectolyase, which is a mixture of pectinase and pectin lyase.

Examples of the oxidoreductases are oxidases such as glucose oxidase, methanol oxidase, bilirubin oxidase, catechol oxidase, laccase, peroxidases such as ligninase and those described in WO-A-97/31090, monooxygenase, dioxygenase such as lipoxygenase and other oxygenases as described in WO-A-99/02632, WO-A-99/02638, WO-A-99/02639 and the cytochrome based enzymatic bleaching systems described in WO-A-99/02641.

The activity of oxidoreductases, in particular the phenol oxidising enzymes in a process for bleaching stains on fabrics and/or dyes in solution and/or antimicrobial treatment can be enhanced by adding certain organic compounds, called enhancers. Examples of enhancers are 2,2'-azo-bis-(3-ethylbenzo-thiazoline-6-sulphonate (ABTS) and Phenothiazine-10-propionate (PTP). More enhancers are described in WO-A-94/12619, WO-A-94/12620 , WO-A-94/12621, WO-A-97/11217, WO-A-99/23887. Enhancers are generally added at a level of 0.01% to 5% by weight of detergent composition.

Builders, polymers and other enzymes as optional ingredients may also be present as found in WO0060045.

Suitable detergency builders as optional ingredients may also be present as found in W00034427.

The invention will now be further illustrated by way of the following non-limiting examples:

### EXAMPLES

### [(MeN4Py)FeCl] C1

The ligand N,N-bis(pyridin- 2-yl-methyl)-1,1-bis(pyridin-2-yl)-1-aminoethane (MeN4py) was prepared as described in EP 0 909 809 A2.

The ligand MeN4Py (33.7 g; 88.5mmoles) was dissolved in 500ml dry methanol. Small portions of FeCl₂.4H₂O (0.95 eq; 16.7 g; 84.0 mmoles) were added, yielding a clear red solution. After addition, the solution was stirred for 30 minutes at room temperature, after which the methanol was removed (rotary-evaporator). The dry solid was ground and 150 ml of ethylacetate was added and the mixture was stirred until a fine red powder was obtained. This powder was washed twice with ethyl acetate, dried in the air and further dried under reduced pressure vacuum at 40 °C. El. Anal. Calc. for [Fe(MeN4py)Cl]Cl.2H₂O: C 53.03; H 5.16; N 12.89; Cl 13.07; Fe 10.01%. Found C 52.29/ 52.03; H 5.05/5.03; N 12.55/12.61; Cl: 12.73/12.69; Fe: 10.06/10.01%.

### Dimethyl 2,6-di-(2-pyridyl)-1-methyl-piperid-4-one-3,5-dicarboxylate (NPy2) (MW: 383.4 g/mol)

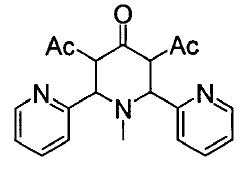

Picolylaldehyde (83.1 mmol; 8 ml) was added drop wise to an ice-bath cooled solution of acetonedicarboxylic acid dimethyl ester (41.55 mmol, 6 ml) in methanol (30 ml), subsequent addition of aqueous (40%) methylamine (41.55 ml, 4.8 ml) yielded an orange red solution. The solution was stirred for 5 min at 0 °C and then cooled to 18 °C. After approximately two days storage at 18 °C large crystals formed in the reaction mixture. The crystals were removed by filtration and washed with cold ethanol and recrystallised from ethanol. Further concentration of the filtrate yielded a further 10% of product. The total yield of the title compound was 12.43 g (78%).
^{**1**}**H-NMR** (CD₂Cl₂) (predominantly enol): 1.70 (s, 3H, -NMe); 3.60; 3.67 (2s, 6H,-OMe); 4.19 (d, J=10Hz, 1H, pipH4); 4.46 (d, J=10Hz, 1H, pipH5); 4.81 (s, 1H, pipH2); 7.10-8.60 (m, 10H, pyHs)

### Dimethyl 2,6-di-(2-pyridine)-1-(pyridin-2-ylmethyl)-piperid-4-one-3,5-dicarboxylate (NPy3) (MW: 460.5 g/mol)

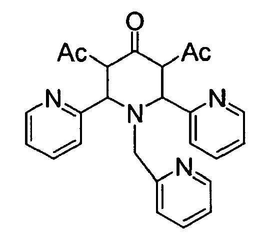

The process for the synthesis of NPy3 is substantially the same as found above for NPy2 except that the following precursors are used: acetonedicarboxylic acid dimethyl ester (0.05 mol; 7.2ml); 2-pyridinaldehyde (0.1 mol; 9.56 ml);
and, picolylamine (0.05 ml; 5.1 ml to yield 19.31 g (84%). ^{**1**}**H-NMR:** (DCCl₃) (predominantly enol): 3.55; 3.81 (s, 6H, - OMe); 3.83 (s, 2H, CH₂-py); 4.29 (d, J=12Hz, 1H, pipH4); 4.81 (d, J=12Hz, 1H, pipH5); 4.89 (s, 1H, pipH2); 7.05-7.78 (m, 9H, pyHs); 8.42-8.48 (m, 2H, pyH6, pyH6); 8.62 (d, J=8Hz), 1H, pyH6)

### Dimethyl 2,4-di-(2-pyridyl) -3,7-dimethyl-3,7-diaza-bicyclo[3.3.1]nonan-9one-1,5-dicarboxylate (N2Py2) (MW:438.5 g/mol)

To a suspension of NPy2 (26.1 mmol; 10g) in 80 ml ethanol was added an aqueous (37%) formaldehyde solution (62.66 mmol, 5.64 ml) followed by an aqueous 40% solution of methylamine (31.33 ml; 3.6 ml). The reaction mixture was then heated at reflux for 5 min after which the reaction mixture was cooled to ambient temperature. After scratching the inside of the vessel holding the reaction mixture white crystals were formed. After filtration of the crystalline product, the product was washed with ethanol and the crystalline product dried under produced pressure to yield 8.61 g (75.3%) of the title compound.
^{**1**}**H-NMR** (CD₂Cl₂): 2.00 (s, 3H, N7-Me); 2.22 (s, 3H, N3-Me); 2.45 (d, J=12Hz, 2H, bisH6ax, bisH8ax); 2.93 (d, J=12Hz, 2H, bisH6eq, bisH8eq); 3.75 (s, 6H, -OMe); 4.67 (s, 2H, bisH2, bisH4); 7.23 (m, 2H, pyH5); 7.80 (t, J=8Hz, 2H, pyH4); 8.07 (d, J=8Hz, 2H, pyH3); 8.49 (d, J=5Hz, 2H, pyH6).

### Dimethyl 2,4-di-(2-pyridyl) -3-methyl-7-(pyridin-2-ylmethyl)-3,7-diaza-bicyclo[3.3.1]nonan-9-one-1,5-dicarboxylate (N2Py3o) (MW: 515.22 g/mol)

2-Aminomethyl-pyridine (4.3 g, 39.7 mmol) and formaldehyde (37% in water) (6.5 mL, 79.4 mmol) were added to a suspension of NPy2 (12.71 g, 33.1 mmol) in 200 mL ethanol. The suspension was stirred under reflux for 30 minutes resulting in a clear brown solution. The solvent was removed under reduced pressure and the remaining solid was crystallised from ethanol to yield the title compound as a white solid (4.2 g, 25 %).
^{**1**}**H-NMR** (300 MHz, CDCl₃) : 1.94 (s, 3H, N-Me), 2.68 (d, 2H, J=12Hz, bisH6ax, bisH8ax-); 3.14 (d, 2H, J=12Hz, bisH6eq, bisH8eq): 3.57 (s, 2H, CH₂-Py), 3.76 (s, 6H, OMe), 4.66 (s, 2H, bisH2, bisH4), 7.09 (t, 2H, J=1.5Hz, Py-H), 7.21 (t, 1H, J=6.0Hz, Py-H), 7.33 (d, 1H, J=7.6Hz, Py-H), 7.50 (t, 2H, J=1.7Hz, Py-H), 7.66 (t, 1H, J=7.5Hz, Py-H), 7.92 (d, 2H, J=7.8Hz, Py-H), 8.45 (d, 2H, J=4.0Hz, Py-H), 8.62 (d, 1H, J=4.8Hz, Py-H).

### Dimethyl 2,4-di-(2-pyridyl)-3-(pyrid-2-ylmethyl)-7-methyl-3,7-diaza-bicyclo[3.3.1]nonan-9-one-1,5-dicarboxylate (N2Py3u) (MW: 515.22 g/mol)

To a suspension of NPy3 (21.79 g, 47.3 mmol) in 250 mL ethanol was added aqueous (40%) methylamine (4.8 mL, 56.7 mmol) and aqueous (37%) formaldehyde (9.2 ml, 113.4 mmol). The suspension was stirred under reflux for 3 h which resulted in a deep brown solution being formed. The solvent was removed under reduced pressure and the resulting green/brown solid was recrystallized from ethanol to yield 6.58 g (27 %) of the title compound as a white solid.
^{**1**}**H-NMR** (300 MHz, CDCl₃): 2.20 (s, 3H, N-Me), 2.56 (d, 2H, J=12Hz, bisH6ax, bisH8ax), 2.98 (d, 2H, J_{HH}=12Hz, bisH6eq, bisH8eq), 3.72 (s, 8H, OMe, CH₂-Py), 5.42 (s, 2H, bisH2, bisH4), 6.76 (d, 1H, J=7.7Hz, Py-H), 6.97 (t, 1H, J=5.7 Hz, Py-H), 7.13 (t, 2H, J=6.0Hz, Py-H), 7.38 (t, 2H, J=7.6Hz, Py-H), 7.68 (t, 2H, J=7.6Hz, Py-H), 8.06 (d, 1H, J=7.6Hz, Py-H), 8.43 (d, 1H, J=4.6Hz, Py-H), 8.47 (d, 2H, J=4.4Hz, Py-H).
Anal. Calcd for C₂₈H₂₉N₅O₅: C 65.23, H 5.67, N 13.58; found: C 64.86, H 5.60, N 13.41.

### Dimethyl 2,4-di-(2-pyridyl) -3,7-bis-(pyridin-2- ylmethyl)-3,7-diaza-bicyclo[3.3.1]nonan-9-one-1,5-dicarboxylate (N2Py4) (MW: 594.7 g/mol)

To a heated solution of NPy3 (32.61 mmol; 15g) in 25 ml THF an aqueous (40 %) formaldehyde (78.3 mmol; 7.0 ml) solution was added drop wise, after which 2-aminomethyl-pyridine (39.1 mmol; 4 ml) was added drop wise resulting in a dark solution. The mixture was further heated for 1 h at 85 °C. After the reaction mixture was cooled a greenish precipitate was formed. The precipitate was then washed with cold ethanol and crystallised from ethanol to yield the title compound, 4.75 g (25%). In some instances no precipitate is formed and in this case it is advisable to remove the THF under reduced pressure to yield a black oil and add 5 ml EtOH. After addition of the EtOH the title compound crystallises out after 3 to 4 hrs.
^{**1**}**H-NMR** (CDCl3): 2.87 (d, J=12Hz, 2H, bisH6ax, bisH8ax); 3.46 (d, J=12Hz, 2H, bisH6eq, bisH8eq), 3.66-3.71 (m, 10H, -OMe, -CH₂-py); 5.35 (s, 2H, bisH2, bisH4); 6.73-8.63 (m s, 20H, pyHs).

Table 1 exemplifies the structures of ligands of the present invention that were used in bleaching experiments.

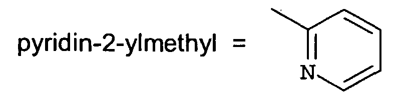

| | |
|---|---|
| 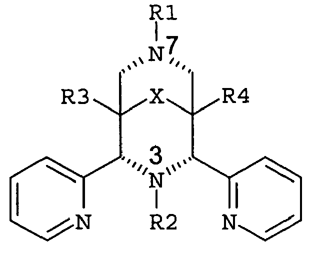 | Ligand |
| R3 = R4 = -C(O)OMe | N2Py4 |
| R1 = R2 = pyridin-2-ylmethyl | |
| R3 = R4 = -C(O)OMe | N2Py2 |
| R1 = R2 = -CH3 | |
| R3 = R4 = -C(O)OMe | N2Py3u |
| R1 = Me | |
| R2 = pyridin-2-ylmethyl | |
| R3 = R4 = -C(O)OMe | N2Py3o |
| R1 = pyridin-2-ylmethyl | |
| R2 = Me | |

### General synthesis of complex from ligand

A solution of 2 mmol metal salt (FeSO₄, FeCl2, CuC12, Fe(ClO₄)₂ etc) in 1 mL methanol was added to a solution of 2 mmol ligand in 1 mL acetonitrile. The clear dark (generally brown for Fe complex and blue for Cu complex) solution was put in a diethylether diffusion bath. After several hours, coloured crystals precipitated from the solution.

### [FeSO₄(N2Py3o)]

(Dimethyl 2,4-di-(2 pyridyl) -3-methyl-7-(pyridin-2-ylmethyl)-3,7-diaza-bicyclo[3.3.1]nonan-9-one-1,5-dicarboxylate)sulfatoiron(II) [C₂₈H₂₉FeN₅O₉S M = 667, 13g/mol]
Anal. Calcd for C₂₈H₂₉FeN₅O₉S: C 47.80, H 4.73, N 9.96; found +2H₂O: C 47.16, H 4.91, N 9.84. FAB⁺MS(nitrobenzylalcohol): 686.1 (MH⁺+H₂O)

### [FeSO₄(N2Py3u)]

((Dimethyl-2,4-di-(2-pyridyl)-3-(pyridin-2-ylmethyl)-7-methyl-3,7-diaza-bicyclo[3.3.1]nonan-9-one-1,5-dicarboxylate)sulfatoiron(II) (M = 667,13g/mol)
Anal. Calcd for C₂₈H₂₉FeN₅O₉S: C 46.61, H 4.89, N 9.71; found +3H₂O: C 47.27, H 4.81, N 9.88. FAB⁺MS(nitrobenzylalcohol): 686.1 (MH⁺+H₂O)

### [FeCl(N2Py3o)]Cl

Chloro(dimethyl 2,4-di-(2-pyridyl) -3-methyl-7-(pyridin-2-ylmethyl)-3,7-diaza-bicyclo[3.3.1]nonan-9-one-1,5-dicarboxylate)iron(II)- chloride hydrate Anal. Calcd for C₂₈H₂₉Cl₂FeN₅O₅:C 49.58, H 4.90, N 10.45; found +2H₂O: C 49.45, H 4.79, N 10.00. FAB⁺MS(nitrobenzylalcohol): 624.1
[FeCl(N2Py3o)·H₂O]

### [Fe(N2Py4)]Cl₂

(Dimethyl-2,4-di-(2-pyridyl)-3,7-bis-(pyridin-2-ylmethyl)-3,7-diaza-bicyclo[3.3.1]nonan-9-one-1,5-dicarboxylate)iron(II)- dichloride hydrate [C₃₃H₃₈Cl₂FeN₆O₆ M = 741.44g/mol], Anal. Calcd for C₃₃H₃₈Cl₂FeN₆O₆: C 53.75, H 4.65, N 11.40; found: C 53.20, H 4.74, N 11.22.
FAB⁺MS(Nitrobenzylalcohol): 683.1 [Fe (N2Py4) ·H₂O]

### [Fe(NCCH₃)₂(triflate) ₂]

The following reaction was performed under anhydrous conditions under argon using standard Schlenck/cannular techniques.

To a cooled stirred mixture of iron powder (5.6 g, 0.1 mol) in acetonitrile (60 ml) trifluoromethanesulfonic acid (0.2 mol, 17.7 ml) was added. After addition, the reaction mixture was heated for 45 min at 90 °C. The reaction mixture was cooled, after which remaining solid material was filtered off. To the remaining solution 40 ml of diethyl ether was slowly added resulting in a white precipitate. The white precipitate was filtered off under argon, and washed with 20 ml of ether. The hygroscopic material was stored under. Yield 31.7% (13.8g).

### [Fe(N2Py2)(triflate)₂] (MW: 792.46 g/mol)

In a water-free system, 0.23 mmol (100 mg) of [Fe (CH₃CN)₂(triflate)₂] and ligand (0.23 mmol; 100 mg) in dry acetonitrile is added. Via slow diffusion of ether into this solution, the crystals with the iron complex are formed. The yield for this procedure is typically 50%. Anal. Calcd for FeC₂₅H₂₉N₄O₁₁S₂F₆ calc 38.91, H 3.51, N 8.40; found: C 38.86, H 3.41, N 8.32.

### Bleaching Experiments

Bleaching results obtained on tomato stains for the different complexes (10 µM) or preformed ligand/iron species (by premixing 2 mM ligand/1mM iron perchlorate in ethanol/water (1/1). The tomato stains were washed with the LAS/buffer system (0.6 g/L NaLAS in 10 mM carbonate buffer) for 30 min at 30 °C in a bottle containing 25 ml of the wash solution. After the wash, cloths were washed with water and dried in a tumble drier till dryness.

The reflectance measurements were obtained using a Minolta™ 3700d spectrophotometer at 460 nm. The difference in reflectance before and after the wash is defined as a ΔR460 value. The bleaching results obtained immediately after drying (t=0) are shown. All values expressed in ΔΔR 460 values (blank, LAS only substracted); typical errors are in the order of 2 points. A higher value means a better bleaching performance.

Table 2: Bleaching results (ΔΔR 460) on tomato oil of the preformed complexes and ligand/iron salt mixtures (active).

**Table 2**

| Active | t=0 |
|---|---|
| N2Py4+Fe(II) | 10 |
| [Fe(N2py3o)Cl]Cl | 24 |
| [Fe(N2py3u)SO4] | 22 |
| N2py3u +Fe(II) | 11 |
| N2py3o +Fe(II) | 20 |
| [Fe(N2py2)Cl₂] | 7 |
| N2py2 + Fe(II) | 1 |

The results in Table 2 show the following:
A good bleaching activity is obtained on tomato oil stains with especially the iron complexes containing N2Py3 ligands (u and o) and to a lesser extent the N2py4 ligand/iron mixture in air bleaching. In all cases the bleaching results are significantly better than the N2py2-containing systems (either Fe complex or ligand/iron salt mixture). It is noteworthy that the ligand in combination with iron salt is effective in air bleaching.

## Claims

1. A bleaching composition comprising:
a) a monomer ligand or transition metal catalyst thereof of a ligand having the formula (I): 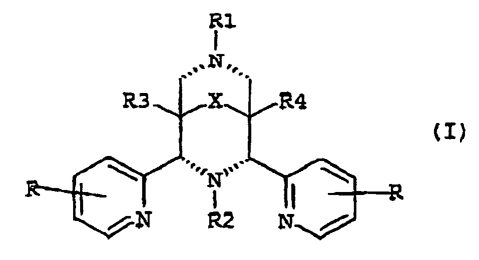 wherein each R is independently selected from: hydrogen, F, Cl, Br, hydroxyl, C1-C4-alkylo-, -NH-CO-H, -NH-CO-C1-C4-alkyl, -NH2, -NH-C1-C4-alkyl, and C1-C4-alkyl;
R1 and R2 are independently selected from:
C1-C4-alkyl,
C6-C10-aryl, and,
a group containing a heteroatom capable of coordinating to a transition metal, wherein at least one of R1 and R2 is the group containing the heteroatom;
R3 and R4 are independently selected from hydrogen, C1-C8 alkyl, C1-C8-alkyl-O-C1-C8-alkyl, C1-C8-alkyl-O-C6-C10-aryl, C6-C₁₀-aryl, C1-C8-hydroxyalkyl, and - (CH2)ₙC(O)OR5
wherein R5 is independently selected from: hydrogen, C1-C4-alkyl, n is from 0 to 4, and mixtures thereof; and,
X is selected from C=O, -[C(R6)₂]_{y}- wherein Y is from 0 to 3 each R6 is independently selected from hydrogen, hydroxyl, C1-C4-alkoxy and C1-C4-alkyl; and,
b) the balance carriers and adjunct ingredients.

2. A bleaching composition according to claim 1, wherein R1 and R2 are both selected from a group containing a heteroatom capable of coordinating to a transition metal.

3. A bleaching composition according to any preceding claim, wherein the group containing the heteroatom is:
a heterocycloalkyl: selected from the group consisting of:
pyrrolinyl; pyrrolidinyl; morpholinyl; piperidinyl; piperazinyl; hexamethylene imine; 1,4-piperazinyl; tetrahydrothiophenyl; tetrahydrofuranyl; tetrahydropyranyl; and oxazolidinyl, wherein the heterocycloalkyl may be connected to the ligand via any atom in the ring of the selected heterocycloalkyl,
a -C1-C6-alkyl-heteracycloalkyl, wherein the heterocycloalkyl of the -C1-C6-heterocycloalkyl is selected from the group consisting of: piperidinyl; piperidine; 1,4-piperazine, tetrahydrothiophene; tetrahydrofuran; pyrrolidine; and tetrahydropyran, wherein the heterocycloalkyl may be connected to the -C1-C6-alkyl via any atom in the ring of the selected heterocycloalkyl,
a -C1-C6-alkyl-heteroaryl, wherein the heteroaryl of the -C1-C6-alkylheteroaryl is selected from the group consisting of: pyridinyl; pyrimidinyl; pyrazinyl; triazolyl; pyridazinyl; 1,3,5-triazinyl; quinolinyl; isoquinolinyl; quinoxalinyl; imidazolyl; pyrazolyl; benzimidazolyl; thiazolyl; oxazolidinyl; pyrrolyl; carbazolyl; indolyl; and
isoindolyl, wherein the heteroaryl may be connected to the -C1-C6-alkyl via any atom in the ring of the selected heteroaryl and the selected heteroaryl is optionally substituted by -C1-C4-alkyl,
a -C0-C6-alkyl-phenol or thiophenol,
a -C2-C4-alkyl-thiol, thioether or alcohol,
a -C2-C4-alkyl-amine, and
a -C2-C4-alkyl-carboxylate.

4. A bleaching composition according to any preceding claim, wherein: each R is the same; and R3 = R4.

5. A bleaching composition according to any preceding claim, wherein R3 and R4 are the same and are -(CH2)ₙC(O)O-C₁-C₄-alkyl.

6. A bleaching composition according to any preceding claim, wherein R3 and R4 are selected from the group consisting of -CH2OH, -C(O)O-C1-C6-alkyl, and phenyl.

7. A bleaching composition according to any proceeding claim, wherein at least one R1 and R2 is a 3-C0-C6-alkyl-pyridin-2-yl-C0-C6-alkyl.

8. A bleaching composition according to any preceding claim, wherein Y = 1

9. A bleaching composition according to any preceding claim, wherein R3 and R4 are -C(O)O-C1-C6-alkyl.

10. A bleaching composition according to any preceding claim, wherein at least one of R1 and R2 is selected from the group consisting of: 3-ethyl-pyridin-2-ylmethyl, pyridin-2-ylmethyl, 3-methyl-pyridin-2-ylmethyl, and 6-amide-pyridin-2-ylmethyl. ,

11. A bleaching composition according to claim 10, wherein at least one of R1 and R2 is pyridin-2-ylmethyl.

12. A bleaching composition according to any preceding claim, wherein both R1 and R2 are pyridin-2-ylmethyl and R is H.

13. A bleaching composition according to any preceding claim, wherein X is C=O.

14. A bleaching composition according to any preceding claim, wherein the bleaching composition comprises the free ligand.

15. A bleaching composition according to claims 1 to 13, wherein the complex is of the general formula (A1):
[MₐLₖXₙ] Yₘ (A1)
in which:
M represents a metal selected from Mn(II)-(III)-(IV)-(V), Cu(I)-(II)-(III), Fe(II)-(III)-(IV)-(V), Co(I)-(II)-(III), Ti(II)-(III)-(IV), V(II)-(III)-(IV)-(V), Mo(II)-(III) - (IV)-(V)-(VI) and W(IV)-(V)-(VI);
X represents a coordinating species selected from any mono, bi or tri charged anions and any neutral molecules able to coordinate the metal in a mono, bi or tridentate manner;
Y represents any non-coordinated counter ion;
a represents an integer from 1 to 10;
k represents an integer from 1 to 10;
n represents an integer from 1 to 10;
m represents zero or an integer from 1 to 20; and
L represents a ligand as defined in claims 1 to 13, or its protonated or deprotonated analogue.

16. A bleaching composition according to claim 15, wherein M represents a metal selected from Fe(II)-(III)-(IV)-(V).

17. A bleaching composition according to claim 16, wherein M represents a metal selected from Fe (II) and Fe(III).

18. A ligand of formula (I) according to claim 1 or a transition metal catalyst thereof with the proviso that the following compounds are excluded:
dimethyl 2,4-di-(2-pyridyl)-3-acetyl-7-methyl-3,7-diaza-bicyclo[3.3.1]nonan-9-one-1,5-dicarboxylate;
dimethyl 2,4-di-(2-pyridyl)--3,7-bis-(pyridin-2-ylmethyl)-3,7-diaza-bicyclo[3.3.1]nonan-9-one-1,5-dicarboxylate;
1,5-bis-(hydroxymethylene)-2,4-di-(2-pyridyl)-3,7-bis-(pyridin-2-ylmethyl)-3,7-diazabicyclo[3.3.1]nonan-9-ol;
dimethyl 2,4-di-(2-pyridyl)-3,7-bis-(pyridin-2-ylethyl)-3,7-diaza-bicyclo[3.3.1]nonan-9-one-1,5-dicarboxylate;
dimethyl 2,4-di-(2-pyridyl)-3-(5-carboxypentyl)-7-methyl-3,7-diaza-bicyclo[3.3.1]nonan-9-one-1,5-dicarboxylate; and,
dimethyl 2,4-di-(2-pyridyl)-3-(2-methoxyethyl)-7-methyl-3,7-diaza-bicyclo[3.3.1]nonan-9-one-1,5-dicarboxylate.

19. A ligand of formula (I) according to claim 1 or a transition metal catalyst thereof, wherein at least one of R1 or R2 is pyridin-2-ylmethyl and the other is selected from -CH3, -C2H5, -C3H7, and -C4H9.

20. A method of bleaching a substrate comprising the step of applying to the substrate, in an aqueous medium, the bleaching composition according any one of claims 1 to 17.

## Patentansprüche

1. Bleichende Zusammensetzung, umfassend:
a) einen Monomerliganden oder Übergangsmetallkatalysator davon von einem Liganden mit der Formel (I): 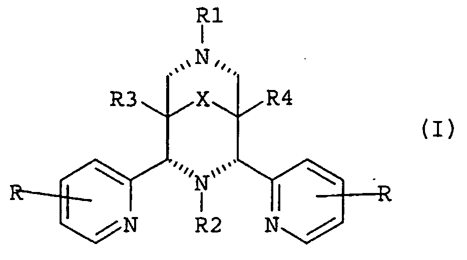 worin jedes R unabhängig ausgewählt ist aus: Wasserstoff, F, Cl, Br, Hydroxyl, C1-C4-AlkylO-, -NH-CO-H, -NH-CO-Cl-C4-Alkyl, -NH2, -NH-C1-C4-Alkyl und C1-C4-Alkyl;
R1 und R2 unabhängig ausgewählt sind aus:
C1-C4-Alkyl,
C6-C10-Aryl, und
einer Gruppe, enthaltend ein Heteroatom, das an ein Übergangsmetall koordinieren kann, wobei mindestens einer von R1 und R2 die das Heteroatom enthaltende Gruppe darstellt;
R3 und R4 unabhängig ausgewählt sind aus Wasserstoff, Cl-C8-Alkyl, C1-C8-Alkyl-O-C1-C8-alkyl, Cl-C8-Alkyl-O-C6-C10-aryl, C6-C10-Aryl, Cl-C8-Hydroxyalkyl und -(CH₂)ₙC(O)OR5,
worin R5 unabhängig ausgewählt ist aus: Wasserstoff, C1-C4-Alkyl, n 0 bis 4 ist, und Gemischen davon; und
X ausgewählt ist aus C=O, -[C(R6)₂]_{y}-, worin Y 0 bis 3 ist, jedes R6 unabhängig ausgewählt ist aus Wasserstoff, Hydroxyl, C1-C4-Alkoxy und C1-C4-Alkyl; und
b) den Rest Träger und Hilfsbestandteile.

2. Bleichende Zusammensetzung nach Anspruch 1, worin R1 und R2 beide ausgewählt sind aus einer Gruppe, enthaltend ein Heteroatom, das mit einem Übergangsmetall koordinieren kann.

3. Bleichende Zusammensetzung nach einem vorangehenden Anspruch, worin die Gruppe, enthaltend das Heteroatom, bedeutet:
ein Heterocycloalkyl: ausgewählt aus der Gruppe, bestehend aus: Pyrrolinyl; Pyrrolidinyl; Morpholinyl; Piperidinyl; Piperazinyl; Hexamethylenimin; 1,4-Piperazinyl; Tetrahydrothiophenyl; Tetrahydrofuranyl; Tetrahydropyranyl und Oxazolidinyl, wobei das Heterocycloalkyl an die Liganden über ein beliebiges Atom in dem Ring des ausgewählten Heterocycloalkyls gebunden sein kann,
ein -C1-C6-Alkyl-heterocycloalkyl, worin das Heterocycloalkyl des -C1-C6-Heterocycloalkyls ausgewählt ist aus der Gruppe, bestehend aus: Piperidinyl; Piperidin; 1,4-Piperazin, Tetrahydrothiophen; Tetrahydrofuran; Pyrrolidin und Tetrahydropyran, wobei das Heterocycloalkyl an das -C1-C6-Alkyl über ein beliebiges Atom in dem Ring von dem ausgewählten Heterocycloalkyl verbunden sein kann,
ein -C1-C6-Alkyl-heteroaryl, worin das Heteroaryl von dem -C1-C6-Alkylheteroaryl ausgewählt ist aus der Gruppe, bestehend aus: Pyridinyl; Pyrimidinyl; Pyrazinyl; Triazolyl; Pyridazinyl; 1,3,5-Triazinyl; Chinolinyl; Isochinolinyl; Chinoxalinyl; Imidazolyl; Pyrazolyl; Benzimidazolyl; Thiazolyl; Oxazolidinyl; Pyrrolyl; Carbazolyl; Indolyl und Isoindolyl, worin das Heteroaryl an das -C1-C6-Alkyl über ein beliebiges Atom in dem Ring von dem ausgewählten Heteroaryl gebunden sein kann, und das ausgewählte Heteroaryl gegebenenfalls substituiert ist mit -C1-C4-Alkyl,
-C0-C6-Alkyl-phenol oder -thiophenol,
-C2-C4-Alkyl-thiol, -thioether oder -alkohol,
-C2-C4-Alkyl-amin und
-C2-C4-Alkyl-carboxylat.

4. Bleichende Zusammensetzung nach einem vorangehenden Anspruch, worin jedes R das gleiche ist und R3 = R4.

5. Bleichende Zusammensetzung nach einem vorangehenden Anspruch, worin R3 und R4 die gleichen sind und -(CH2)ₙC(O)O-C1-C4-Alkyl bedeuten.

6. Bleichende Zusammensetzung nach einem vorangehenden Anspruch, worin R3 und R4 ausgewählt sind aus der Gruppe, bestehend aus -CH2OH, -C(O)O-C1-C6-Alkyl und Phenyl.

7. Bleichende Zusammensetzung nach einem vorangehenden Anspruch, worin mindestens einer von R1 und R2 ein 3-C0-C6-Alkyl-pyridin-2-yl-C0-C6-alkyl ist.

8. Bleichende Zusammensetzung nach einem vorangehenden Anspruch, worin Y = 1.

9. Bleichende Zusammensetzung nach einem vorangehenden Anspruch, worin R3 und R4 -C(O)O-C1-C6-Alkyl sind.

10. Bleichende Zusammensetzung nach einem vorangehenden Anspruch, worin mindestens einer von R1 und R2 ausgewählt ist aus der Gruppe, bestehend aus: 3-Ethyl-pyridin-2-yl-methyl, Pyridin-2-ylmethyl, 3-Methyl-pyridin-2-ylmethyl und 6-Amid-pyridin-2-ylmethyl.

11. Bleichende Zusammensetzung nach Anspruch 10, worin mindestens einer von R1 und R2 Pyridin-2-ylmethyl ist.

12. Bleichende Zusammensetzung nach einem vorangehenden Anspruch, worin beide R1 und R2 Pyridin-2-ylmethyl sind und R H darstellt.

13. Bleichende Zusammensetzung nach einem vorangehenden Anspruch, worin X die C=O darstellt.

14. Bleichende Zusammensetzung nach einem vorangehenden Anspruch, worin die bleichende Zusammensetzung einen freien Liganden umfasst.

15. Bleichende Zusammensetzung nach Anspruch 1-13, worin der Komplex die allgemeine Formel (A1) aufweist
[MₐLₖXₙ]Yₘ (A1)
worin
M ein Metall, ausgewählt aus: Mn(II)-(III)-(IV)-(V), Cu(I)-(II)-(III), Fe(II)-(III)-(IV)-(V), Co(I)-(II)-(III), Ti(II)-(III)-(IV), V(II)-(III)-(IV)-(V), Mo(II)-(III)-(IV)-(V)-(VI) und W(IV)-(V)-(VI), wiedergibt;
X eine koordinierende Spezies, ausgewählt aus beliebigen ein-, zwei- oder dreifach geladenen Anionen, und beliebigen neutralen Molekülen, die an das Metall in einer 1-, 2- oder 3-zähnigen Weise koordinieren können, wiedergibt;
Y beliebiges, nicht koordiniertes Gegenion wiedergibt;
a eine ganze Zahl von 1 bis 10 wiedergibt;
k eine ganze Zahl von 1 bis 10 wiedergibt;
n eine ganze Zahl von 1 bis 10 wiedergibt;
m 0 oder eine ganze Zahl von 1 bis 20 wiedergibt und
L einen Liganden, wie in Ansprüchen 1 bis 13 definiert, wiedergibt, oder dessen protoniertes oder deprotoniertes Analoges.

16. Bleichende Zusammensetzung nach Anspruch 15, wobei M ein Metall, ausgewählt aus Fe(II)-(III)-(IV)-(V), wiedergibt.

17. Bleichende Zusammensetzung nach Anspruch 16, wobei M ein Metall, ausgewählt aus Fe(II) und Fe(III), wiedergibt.

18. Ligand der Formel (I) nach Anspruch 1 oder ein Übergangsmetallkatalysator davon, mit der Maßgabe, dass die nachstehenden Verbindungen ausgeschlossen sind:
2,4-Di-(2-pyridyl)-3-acetyl-7-methyl-3,7-diaza-bicyclo-[3.3.1]nonan-9-on-1,5-dicarbonsäuredimethylester;
2,4-Di-(2-pyridyl)-3,7-bis-(pyridin-2-ylmethyl)-3,7-diazabicyclo[3.3.1]nonan-9-on-1,5-dicarbonsäuredimethylester;
1,5-Bis-(hydroxymethylen)-2,4-di-(2-pyridyl)-3,7-bis-(pyridin-2-ylmethyl)-3,7-diazabicyclo[3.3.1]nonan-9-ol;
2,4-Di-(2-pyridyl)-3,7-bis-(pyridin-2-ylethyl)-3,7-diazabicyclo[3.3.1]nonan-9-on-1,5-dicarbonsäuredimethylester;
2,4-Di-(2-pyridyl)-3-(5-carboxypentyl)-7-methyl-3,7-diazabicyclo[3.3.1]nonan-9-on-1,5-dicarbonsäuredimethylester und
2,4-Di-(2-pyridyl)-3-(2-methoxyethyl)-7-methyl-3,7-diazabicyclo-[3.3.1]-nonan-9-on-1,5-dicarbonsäuredimethylester.

19. Ligand der Formel (I) nach Anspruch 1 oder ein Übergangsmetallkatalysator davon, wobei mindestens einer von R1 oder R2 Pyridin-2-ylmethyl darstellt und der andere ausgewählt ist aus: -CH3, -C2H5, -C3H7 und -C4H9.

20. Verfahren zum Bleichen eines Substrats, umfassend den Schritt des Anwendens der bleichenden Zusammensetzung nach einem der Ansprüche 1 bis 17 in einem wässrigen Medium auf das Substrat.

## Revendications

1. Composition de blanchiment comprenant :
a) un ligand monomère ou un catalyseur de celui-ci à base de métal de transition, ce ligand ayant la formule (I) : 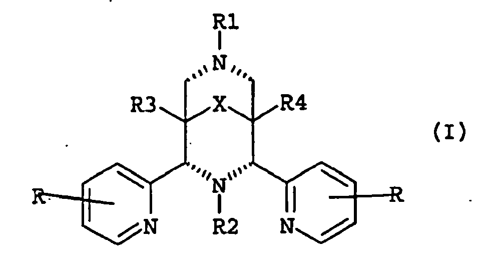 dans laquelle chaque R est indépendamment choisi parmi : un atome d'hydrogène, F, Cl, Br, un groupe hydroxyle, (alkyle en C₁ à C₄)-O-, -NH-CO-H, -NH-CO-(alkyle en C₁ à C₄), -NH₂, -NH-(alkyle en C₁ à C₄) et alkyle en C₁ à C₄ ;
R1 et R2 sont choisis indépendamment parmi :
- un groupe alkyle en C₁ à C₄,
- un groupe aryle en C₆ à C₁₀, et
- un groupe contenant un hétéroatome capable de se coordonner à un métal de transition, dans laquelle au moins un de R1 et R2 représente le groupe contenant l'hétéroatome ;
R3 et R4 sont choisis indépendamment parmi un atome d'hydrogène, un groupe alkyle en C₁ à C₈, (alkyle en C₁ à C₈)-O-(alkyle en C₁ à C₈), (alkyle en C₁ à C₈)-O-(aryle en C₆ à C₁₀), aryle en C₆ à C₁₀, hydroxyalkyle en C₁ à C₈ et - (CH₂)ₙC(O)OR5
dans laquelle R5 est indépendamment choisi parmi : un atome d'hydrogène, un groupe alkyle en C₁ à C₄, n vaut de 0 à 4, et leurs mélanges ; et, X est choisi parmi un groupe C=O, -[C(R6)₂]_{y}- dans lequel Y vaut de 0 à 3
chaque R6 est indépendamment choisi parmi un atome d'hydrogène, un groupe hydroxyle, alcoxy en C₁ à C₄ et alkyle en C₁ à C₄ ; et,
b) le complément d'ingrédients de type additif et véhicules.

2. Composition de blanchiment selon la revendication 1, dans laquelle R1 et R2 sont tous deux choisis dans un groupe contenant un hétéroatome capable de se coordonner à un métal de transition.

3. Composition de blanchiment selon l'une quelconque des revendications précédentes, dans laquelle le groupe contenant l'hétéroatome est :
- un groupe hétérocycloalkyle, choisi dans le groupe constitué par les groupes : pyrrolinyle ; pyrrolidinyle ; morpholinyle ; pipéridinyle ; pipérazinyle ; hexaméthylèneimine ; 1,4-pipérazinyle ; tétrahydrothiophényle ; tétrahydrofuranyle ; tétrahydropyranyle ; et oxazolidinyle, dans laquelle le groupe hétérocycloalkyle peut être lié au ligand par l'intermédiaire de tout atome du cycle du groupe hétérocycloalkyle choisi,
- un groupe -(alkyle en C₁ à C₆)-hétérocycloalkyle, dans lequel le groupe hétérocycloalkyle du groupe -(alkyle en C₁ à C₆)hétérocycloalkyle est choisi dans le groupe constitué par les groupes : pipéridinyle ; pipéridine ; 1,4-pipérazine ; tétrahydrothiophène ; tétrahydrofuranne ; pyrrolidine ; et tétrahydropyranne, dans lequel le groupe hétérocycloalkyle peut être lié au groupe -alkyle en C₁ à C₆ par l'intermédiaire de tout atome du cycle du groupe hétérocycloalkyle choisi,
- un groupe -(alkyle en C₁ à C₆)-hétéroaryle, dans lequel le groupe hétéroaryle du groupe -(alkyle en C₁ à C₆)-hétéroaryle est choisi dans le groupe constitué par les groupes : pyridinyle ; pyrimidinyle ; pyrazinyle ; triazolyle ; pyridazinyle ; 1,3,5-triazinyle ; quinoléinyle ; isoquinoléinyle ; quinoxalinyle ; imidazolyle ; pyrazolyle ; bénzimidazolyle ; thiazolyle ; oxazolidinyle ; pyrrolyle ; carbazolyle ; indolyle ; et isoindolyle, dans laquelle le groupe hétéroaryle peut être lié au groupe -alkyle en C₁ à C₆ par l'intermédiaire de tout atome du cycle du groupe hétéroaryle choisi et le groupe hétéroaryle choisi est éventuellement substitué par un groupe -alkyle en C₁ à C₄,
- un groupe (alkyle en C₀ à C₆)-phénol ou thiophénol,
- un groupe (alkyle en C₂ à C₄)-thiol, thioéther ou alcool,
- un groupe (alkyle en C₂ à C₄)-amine, et
- un groupe (alkyle en C₂ à C₄)-carboxylate.

4. Composition de blanchiment selon l'une quelconque des revendications précédentes, dans laquelle : chaque R est identique ; et R3 = R4.

5. Composition de blanchiment selon l'une quelconque des revendications précédentes, dans laquelle R3 et R4 sont identiques et représentent un groupe -(CH₂)ₙC(O)O-(alkyle en C₁ à C₄).

6. Composition de blanchiment selon l'une quelconque des revendications précédentes, dans laquelle R3 et R4 sont choisis dans le groupe constitué par les groupes CH₂OH, -C(O)O-(alkyle en C₁ à C₆) et phényle.

7. Composition de blanchiment selon l'une quelconque des revendications précédentes, dans laquelle au moins un de R1 et de R2 représente un groupe 3-(alkyle en C₀ à C₆)pyridin-2-yl-(alkyle en C₀ à C₆).

8. Composition de blanchiment selon l'une quelconque des revendications précédentes, dans laquelle Y = 1.

9. Composition de blanchiment selon l'une quelconque des revendications précédentes, dans laquelle R3 et R4 représentent un groupe -C(O)O-(alkyle en C₁ à C₆).

10. Composition de blanchiment selon l'une quelconque des revendications précédentes, dans laquelle au moins un de R1 et de R2 est choisi dans le groupe constitué par les groupes : 3-éthyl-pyridin-2-ylméthyle, pyridin-2-ylméthyle, 3-méthyl-pyridin-2-ylméthyle et 6-amide-pyridin-2-ylméthyle.

11. Composition de blanchiment selon la revendication 10, dans laquelle au moins un de R1 et de R2 représente le groupe pyridin-2-ylméthyle.

12. Composition de blanchiment selon l'une quelconque des revendications précédentes, dans laquelle R1 et R2 représentent tous deux un groupe pyridin-2-ylméthyle et R représente un atome d'hydrogène.

13. Composition de blanchiment selon l'une quelconque des revendications précédentes, dans laquelle X représente un groupe C=O.

14. Composition de blanchiment selon l'une quelconque des revendications précédentes, dans laquelle la composition de blanchiment comprend le ligand libre.

15. Composition de blanchiment selon les revendications 1 à 13, dans laquelle le complexe présente la formule générale (A1) :
[MₐLₖXₙ]Yₘ (A1)
dans laquelle :
- M représente un métal choisi parmi Mn(II) - (III) - (IV) - (V), Cu(I) - (II) - (III), Fe(II) - (III) - (IV) - (V), Co(I) - (II) - (III), Ti(II) - (III) - (IV), V(II) - (III) - (IV) - (V), Mo(II) - (III) - (IV) - (V) - (VI) et W(IV) - (V) - (VI) ;
- X représente une espèce de coordination choisie parmi tout anion mono-, bi- ou tri- chargé et toute molécule neutre capable de se coordonner au métal de manière mono-, bi- ou tri-dentée ;
- Y représente tout contre-ion non coordonné ;
- a représente un nombre entier valant de 1 à 10 ;
- k représente un nombre entier valant de 1 à 10 ;
- n représente un nombre entier valant de 1 à 10 ;
- m représente zéro ou un nombre entier valant de 1 à 20 ; et
- L représente un ligand tel que défini dans les revendications 1 à 13 ou son analogue protoné ou déprotoné.

16. Composition de blanchiment selon la revendication 15, dans laquelle M représente un métal choisi parmi Fe(II) - (III) - (IV) - (V).

17. Composition de blanchiment selon la revendication 16, dans laquelle M représente un métal choisi parmi Fe(II) et Fe(III).

18. Ligand de formule (I) selon la revendication 1 ou catalyseur à base de métal de transition de celui-ci, à condition que les composés suivants soient exclus :
le 2,4-di-(2-pyridyle)-3-acétyl-7-méthyl-3,7-diaza-bicyclo[3.3.1]nonan-9-one-1,5-dicarboxylate de diméthyle ; le 2,4-di-(2-pyridyle)-3,7-bis-(pyridin-2-ylméthyle)-3,7-diaza-bicyclo[3.3.1]nonan-9-one-1,5-dicarboxylate de diméthyle ; le 1,5-bis-(hydroxyméthylène)-2,4-di-(2-pyridyle)-3,7-bis-(pyridin-2-ylméthyle)-3,7-diaza-bicyclo[3.3.1]nonan-9-ol ; le 2,4-di-(2-pyridyle)-3,7-bis-(pyridin-2-yléthyle)-3,7-diaza-bicycio[3.3.1]nonan-9-one-1,5-dicarboxylate de diméthyle ; le 2,4-di-(2-pyridyle)-3-(5-carboxypentyle)-7-méthyl-3,7-diaza-biayclo[3.3.1]nonan-9-one-1,5-dicarboxylate de diméthyle ; et le 2,4-di-(2-pyridyle)-3-(2-méthoxyéthyle)-7-méthyl-3,7-diaza-bicyclo[3.3.1]nonan-9-one-1,5-dicarboxylate de diméthyle.

19. Ligand de formule (I) selon la revendication 1 ou catalyseur à base de métal de transition de celui-ci, dans lequel au moins un de R1 ou de R2 représente le pyridin-2-ylméthyle et l'autre est choisi parmi les groupes -CH₃, -C₂H₅, -C₃H₇ et -C₄H₉.

20. Procédé de blanchiment d'un substrat comprenant l'étape consistant à appliquer au substrat, dans un milieu aqueux, la composition de blanchiment selon l'une quelconque des revendications 1 à 17.
